# EUROPEAN PATENT SPECIFICATION

(11) **EP 1 349 958 B1**
(45) Date of publication and mention of the grant of the patent: **03.06.2009**
(21) Application number: 01971326.2
(22) Date of filing: 24.09.2001
(51) Int. Cl.: C12Q 1/68

(54) **PROBES, PROBE SETS, METHODS AND KITS PERTAINING TO THE DETECTION, IDENTIFICATION AND/OR ENUMERATION OF BACTERIA**
SONDEN, SONDENSÄTZEN,VERFAHREN UND KITS ZUR NACHWEIS, IDENTIFIZIERUNG UND/ODER DIE ZÄHLUNG VON BAKTERIEN
SONDES, JEU DE SONDES, TECHNIQUES ET KITS CONCERNANT LA DETECTION, L'IDENTIFICATION ET/OU LE DENOMBREMENT DE BACTERIES

(30) Priority: 26.09.2000 US 235952 P
(43) Date of publication of application: 08.10.2003
(73) Proprietor: Boston Probes, Inc., Bedford, MA 01730 (US)
(72) Inventor: HYLDIG-NIELSEN, Jens, J., Holliston, MA 01746 (US)
(74) Representative: Smart, Peter John
(86) International application number: PCT/US2001/029874
(87) International publication number: WO 2002/027036

(56) References cited:
- WO-A-89/05359
- WO-A-98/15648
- WO-A-99/02730
- CHEMICAL ABSTRACTS, vol. 123, no. 1, 3 July 1995 (1995-07-03) Columbus, Ohio, US; abstract no. 2735, NUNOFUJI, SATOSHI ET AL: "Methods for detecting RNA using two probes that are complementary to two adjacent domains of targets" XP002232945 & JP 07 039398 A (NIPPON FLOUR MILLS, JAPAN;NAT FEDERATION AGRIC COOP ASS) 10 February 1995 (1995-02-10)
- ZHU Q ET AL: "Detection of Salmonella typhi by polymerase chain reaction." JOURNAL OF APPLIED BACTERIOLOGY, vol. 80, no. 3, 1996, pages 244-251, XP002096352 ISSN: 0021-8847
- NORDENTOFT STEEN ET AL: "Evaluation of a fluorescence-labelled oligonucleotide probe targeting 23S rRNA for in situ detection of Salmonella serovars in paraffin-embedded tissue sections and their rapid identification in bacterial smears" JOURNAL OF CLINICAL MICROBIOLOGY, WASHINGTON, DC, US, vol. 35, no. 10, 1997, pages 2642-2648, XP002162308 ISSN: 0095-1137
- CHRISTENSEN H ET AL: "PHYLOGENETIC RELATIONSHIPS OF SALMONELLA BASED ON rRNA SEQUENCES" INTERNATIONAL JOURNAL OF SYSTEMATIC BACTERIOLOGY, SOCIETY FOR GENERAL MICROBIOLOGY, READING, GB, vol. 48, no. 2, April 1998 (1998-04), pages 605-610, XP009003276 ISSN: 0020-7713
- DATABASE EMBL [Online] EBI; 16 March 1998 (1998-03-16) NORDENTOFT S.: "Salmonella typhimurium 23S ribosomal RNA gene, partial sequence." Database accession no. U88706 XP002225717
- DATABASE EMBL [Online] EBI; 16 March 1998 (1998-03-16) NORDENTOFT S. ET AL.: "Citrobacter amalonaticus 23S ribosomal RNA gene, partial sequence." Database accession no. U88707 XP002225718
- DATABASE EMBL [Online] ebi; 22 July 1998 (1998-07-22) CHRISTENSEN H.: " Salmonella enterica 1,4,[5],12,:i:1,2 23S ribosomal RNA (rrl) gene, complete sequence" Database accession no. U77920 XP002225719
- BOGUSH M L ET AL: "Identification and localization of differences between Escherichia coli and Salmonella typhimurium genomes by suppressive subtractive hybridization." MOLECULAR AND GENERAL GENETICS, vol. 262, no. 4-5, December 1999 (1999-12), pages 721-729, XP002233081 ISSN: 0026-8925

### SEQUENCE LISTING

<110> Boston Probes, Inc.
<120> Probes, Probe Sets, Methods And Kits Pertaining To The Detection, Identification, And/Or Enumeration Of Bacteria
<130> BP0001-PCT
<140>
   <141>
<150> 60/235,952
   <151> 2000-09-26
<160> 3
<170> PatentIn Ver. 2.1
<210> 1
   <211> 15
   <212> DNA
   <213> Artificial Sequence
<220>
   <223> Description of Combined DNA/RNA Molecule:Probing Nucleobase Sequence
<220>
   <223> Description of Artificial Sequence:Probing Nucleobase Sequence
<400> 1
   gtgttaaagt gaacc 15
<210> 2
   <211> 15
   <212> DNA
   <213> Artificial Sequence
<220>
   <223> Description of Combined DNA/RNA Molecule:Probing Nucleobase Sequence
<220>
   <223> Description of Artificial Sequence:Probing Nucleobase Sequence
<400> 2
   agccttgatt ttccg 15
<210> 3
   <211> 15
   <212> DNA
   <213> Artificial Sequence
<220>
   <223> Description of Combined DNA/RNA Molecule:Probing Nucleobase Sequence
<220>
   <223> Description of Artificial Sequence:Probing
   Nucleobase Sequence
<400> 3
   acctacgtgt cagcg 15

## Description

### Background of the Invention:

### 1. Technical Field

This invention is related to the field of probe-based detection, analysis and/or quantitation of organisms in general and in particular, bacteria of the Salmonella genus.

### 2. Background Art

Nucleic acid hybridization is a fundamental process in molecular biology. Probe-based assays are useful in the detection, quantitation and/or analysis of nucleic acids. Nucleic acid probes have long been used to analyze samples for the presence of nucleic acid from bacteria, fungi, virus or other organisms and are also useful in examining genetically-based disease states or clinical conditions of interest. Nonetheless, probe-based assays have been slow to achieve commercial success. This lack of commercial success is, at least partially, the result of difficulties associated with specificity, sensitivity and/or reliability.

Hybridization assays hold promise as a means to screen large numbers of samples for conditions of interest. In practice, however, it is often difficult to multiplex a hybridization assay given the requirement that each of the many very different probes in the assay must exhibit a very high degree of specificity for a specific target nucleic acid under the same or similar conditions of stringency. Given the difficulties in specificity, sensitivity and reliability of nucleic acid probes in assays designed to detect a single target nucleic acid, sensitive and reliable methods for the multiplex analysis of samples have been particularly elusive.

Despite its name, Peptide Nucleic Acid (PNA) is neither a peptide, a nucleic acid nor is it an add. Peptide Nucleic Acid (PNA) is a non-naturally occurring polyamide that can hybridize to nucleic acid (DNA and RNA) with sequence specificity (See: United States Patent No. 5,539,082 and Egholm et al., Nature 365: 566-568 (1993)). Being a non-naturally occurring molecule, unmodified PNA is not known to be a substrate for the enzymes that are known to degrade peptides or nucleic acids. Therefore, PNA should be stable in biological samples, as well as have a long shelf-life. Unlike nucleic acid hybridization, which is very dependent on ionic strength, the hybridization of a PNA with a nucleic acid is fairly independent of ionic strength and is favored at low ionic strength, conditions that strongly disfavor the hybridization of nucleic acid, to nucleic acid (Egholm et al., Nature, at p. 567). The effect of ionic strength on the stability and conformation of PNA complexes has been extensively investigated (Tomac et al., J. Am. Chem. Soc. 118:55 44-5552 (1996)). Sequence discrimination is more efficient for PNA recognizing DNA than for DNA recognizing DNA (Egholm et al., Nature, at p. 566). However, the advantages in point mutation discrimination with PNA probes, as compared with DNA probes, in a hybridization assay, appears to be somewhat sequence dependent (Nielsen et al., Anti-Cancer Drug Design 8:53-65, (1993) and Weiler et al., Nucl. Acids Res. 25: 2792-2799 (1997)).

Though they hybridize to nucleic acid with sequence specificity (See: Egholm et al., Nature, at p. 567), PNAs have been slow to achieve commercial success at least partially due to cost, sequence specific properties/problems associated with solubility and self-aggregation (See: Bergman, F., Bannwarth, W. and Tam, S., Tett. Lett. 36:6823-6826 (1995), Haaima, G., Lohse, A., Buchardt, O. and Nielsen, P.E., Angew. Chem. Int. Ed. Engl. 35:1939-1942 (1996) and Lesnik, E., Hassman, F., Barbeau, J., Teng, K. and Weiler, K., Nucleosides & Nucleotides 16:1775-1779 (1997) at p 433, col. 1, In. 28 through coL 2, In. 3) as well as the uncertainty pertaining to non-specific interactions that might occur in complex systems such as a cell (See: Good, L. et al., Antisense & Nucleic Acid Drug Development 7:431-437 (2997)). Because of their unique properties, it is clear that PNA is not the equivalent of a nucleic acid in either structure or function. Consequently, PNA probes need to be evaluated for performance to thereby confirm whether they can be used to specifically and reliably detect a particular nucleic add target sequence, particularly when the target sequence exists in a complex sample such as a cell, tissue or organism.

### Disclosure Of The Invention

### 1. Summary:

This invention is directed to a method for specifically detecting, identifying and/ or quantitating (enumerating) bacteria of the Salmonella genus, in a sample, among other detectable organisms with which probes for the Salmonella bacteria will cross react. The method comprises contacting the sample, under suitable hybridization conditions, with one or more detectable probes suitable for detecting, identifying and/or quantitating bacteria of the Salmonella genus as well as with one or more independently detectable probes that are specie for one or more other detectable organisms with which the detectable probes for the Salmonella bacteria will cross react, wherein the independently detectable probes do not themselves cross react with the Salmonella bacteria. Hybridization, under suitable hybridization conditions, of the probing nucleobase sequence of the detectable and independently detectable probes to their target sequences is then determined by directly or indirectly detecting the probe/target sequence complexes in the sample. The presence, absence and/or number of organisms that are stained with both the detectable probes and the independently detectable probes is then determined so that those bacteria that are stained with only the detectable probe or probes can be specifically determined for purposes of specific detection, identification and/or quantitation of the Salmonella bacteria in the sample. In this manner, there is a correction made for the non-specific character of the probe or probes chosen for specific detection of the Salmonella bacteria.

Because this method can be used to specifically determine the organisms that exhibit cross reactivity with the Salmonella bacteria of interest, this invention facilitates the use of probes of less than optimal specificity in an assay for specifically determining Salmonella bacteria in a sample of interest.

Even if the probe sequence or sequences chosen are not, or cannot be, made specific for the Salmonella bacteria sought to be determined, it is possible to subtract out the organisms that exhibit cross reactivity and thereby achieve the desired specificity. The only requirement is that the independently detectable probe or probes chosen to detect the troublesome organism or organisms do not themselves exhibit cross reactivity with the Salmonella bacteria of interest.

### 2. Detailed Description Of The Preferred Embodiments Of The Invention

### I. Definitions:

a. As used herein, the term "nucleobase" means those naturally occurring and those non naturally occurring heterocyclic moieties commonly known to those who utilize nucleic acid technology or utilize peptide nucleic acid technology to thereby generate polymers that can sequence specifically bind to nucleic acids.
b. As used herein, the term "nucleobase sequence" means any segment of a polymer that comprises nucleobase containing subunits. Non-limiting examples of suitable polymers or polymers segments include oligodeoxynucleotides, oligoribonucleotides, peptide nucleic acids, nucleic acid analogs, nucleic acid mimics, and/or chimeras.
c. As used herein, the term "target sequence" means the nucleobase sequence that is to be detected in an assay.
d. As used herein, the term "probe" means a polymer (e.g. a DNA, RNA, PNA, chimera or linked polymer) having a probing nucleobase sequence that is designed to sequence specifically hybridize to a target sequence of a target molecule of an organism of interest.
e. As used herein, "strained" means that the individual bacteria are marked for detection, identification and/or quantitation.
f. As used herein, the term "peptide nucleic acid" or "PNA" means any oligomer, linked polymer or chimeric oligomer, comprising two or more PNA subunits (residues), including any of the polymers referred to or claimed as peptide nucleic adds in United States Patent Nos. 5,539,082, 5,527,675, 5,623,049, 5,714,331, 5,736,336, 5,773,571, 5,786,461, 5,837,459, 5,891,625, 5,972,610 and 5,986,053). The term "peptide nucleic acid" or "PNA" shall also apply to polymers comprising two or more subunits of those nucleic acid mimics described in the following publications: Diderichsen et al., Tett. Lett. 37: 475-478 (1996); Fujii et al., Bioorg. Med. Chem. Lett. 7: 637-627 (1997); Jordan et al., Bioorg. Med. Chem. Lett. 7: 687-690 (1997); Krotz et al., Tett. Lett. 36: 6941-6944 (1995); Lagriffoul et al., Bioorg. Med. Chem. Lett. 4: 1081-1082 (1994); Lowe et al., J. Chem. Soc. Perkin Trans. 1, (1997) 1: 539-546; Lowe et al., J. Chem. Soc. Perkin Trans. 11: 547-554 (1997); Lowe et al., J. Chem. Soc. Perkin Trans. 11:5 55-560 (1997); Petersen et al., Bioorg. Med. Chem. Lett. 6: 793-796 (1996); Diederichsen, U., Bioorganic & Med. Chem. Lett., 8: 165-168 (1998); Cantin et al, Tett. Lett., 38: 4211-4214 (1997); Ciapetti et al., Tetrahedron, 53: 1167-1176 (1997); Lagriffoule et al., Chem. Eur. J., 3: 912-919 (1997) and the Peptide-Based Nucleic Acid Mimics (PENAMs) of Shah et al as disclosed in WO96/04000.
   In preferred embodiments, a PNA is a polymer comprising two or more subunits of the formula: 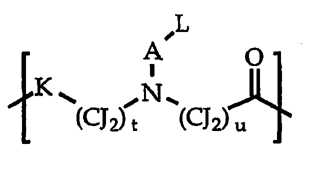 wherein, each J is the same or different and is selected from the group consisting of H, R¹,OR¹ SR¹_{,} NHR¹, NR¹₂ F, C1, Br and I. Each K is the same or different and is selected from the group consisting of O S, NH and NR¹. Each R¹ is the same or different and is an alkyl group having one to five carbon atoms that may optionally contain a heteroatom or a substituted or unsubstituted aryl group. Each A is selected from the group consisting of a single bond, a group of the formula; -(CJ₂)₈- and a group of the formula; -(CJ₂)₈,C(O)-, wherein, J is defined above and eachs is an integer from one to five. The integer t is 1 or 2 and the integer u is 1 or 2. Each L is the same or different and is independently selected from the group consisting of J, adenine, cytosine, guanine, thymine, uridine, 5-methylcytosine, 2-aminopurine, 2-amino-6-chloropurine, 2,6-diaminopurine, hypoxanthine, pseudoisocytosine, 2-thiouracil, 2-thiothymidine, other naturally occurring nucleobase analogs, other non-naturally occurring nucleobases, substituted and unsubstituted aromatic moieties, biotin, fluorescein and dabcyl. In the most preferred embodiment, a PNA subunit consists of a naturally occurring or non-naturally occurring nucleobase attached to the aza nitrogen of the N-[2-(aminoethyl)]glycine backbone through a methylene carbonyl linkage.
g. As used herein, the terms "label" and "detectable moiety" are interchangeable and shall refer to moieties that can be attached to a probe, antibody or antibody fragment to thereby render the probe, antibody or antibody fragment detectable by an instrument or method.
h. As used herein, the term "chimera" or "chimeric oligomer" means an oligomer comprising two or more linked subunits that are selected from different classes of subunits. For example, a PNA/DNA chimera would comprise at least two PNA subunits linked to at least one 2'-deoxynbonucleic acid subunit (For exemplary methods and compositions related to PNA/DNA chimera preparation See: WO96/40709). Exemplary component subunits of the chimera are selected from the group consisting of PNA subunits, naturally and non-naturally occurring amino acid subunits, DNA subunits, RNA subunits and subunits of analogues or mimics of nucleic adds.
i. As used herein, the term "linked polymer" means a polymer comprising two or more polymer segments that are linked by a linker. The polymer segments that are linked to form the linked polymer are selected from the group consisting of an oligodeoxynucleotide (DNA), an oligoribonucleotide (PNA), a peptide, a polyamide, a peptide nucleic acid (PNA) and a chimera.

### II. General:

### Nucleic Acid Synthesis and Modification

Nucleic acid oligomer (oligonucleotide and oligoribonucleotide) synthesis has become routine. For a detailed description of nucleic acid synthesis please see Gait, M. J., Oligonucleotide Synthesis: a Practical Approach. IRL Press, Oxford Engl*and.* Those of ordinary skill in the art will recognize that both labeled or unlabeled oligonucleotides (DNA, RNA and synthetic analogues thereof) are readily available. They can be synthesized using commercially available instrumentation and reagents or they can be purchased from commercial vendors of custom manufactured oligonucleotides. Patents that discuss various compositions, supports and methodologies for the synthesis and labeling of nucleic acids include: 5,476,925, 5,453,496, 5,446,137, 5,419,966, 5,391,723, 5,391,667, 5,380,833, 5,348,868, 5,281,701, 5,278,302, 5,262,530, 5,243,038, 5,218,103, 5,204,456, 5,204,455, 5,198, 540, 5,175,209, 5,164,491, 5,112,962, 5,071,974, 5,047,524, 4,980,460, 4,923,901, 4,786,724, 4,725,677, 4,659,774, 4,500,707, 4,458,066, and 4,415,732.

### PNA Synthesis:

Methods for the chemical assembly of PNAs are well known (See: Patent Nos. 5,539,082, 5,527,675, 5,623,049, 5,714,331, 5,736,336, 5,773,571, 5,786,461, 5,837,459, 5,891,625, 5,972,610 and 5,986,053. Chemicals and instrumentation for the support bound automated chemical assembly of peptide nucleic acids are now commercially available. Both labeled and unlabeled PNA oligomers are likewise available from commercial vendors of custom PNA oligomers. Chemical assembly of a PNA is analogous to solid phase peptide synthesis, wherein at each cycle of assembly the oligomer possesses a reactive alkyl amino terminus that is condensed with the next synthon to be added to the growing polymer. Because standard peptide chemistry is utilized, natural and non-natural amino acids are routinely incorporated into a PNA oligomer. Because a PNA is a polyamide, it has a C-terminus (carboxyl terminus) and an N-terminus (amino terminus). For the purposes of the design of a hybridization probe suitable for antiparallel binding to the target sequence (the preferred orientation), the N-terminus of the probing nucleobase sequence of the PNA probe is the equivalent of the 5'-hydroxyl terminus of an equivalent DNA or RNA oligonucleotide.

### PNA Labeling:

Preferred non-limiting methods for labeling PNAs are described in US 6,110,676, WO99/22018, WO99/21881, WO99/49293 and WO99/37670, the examples section of this specification or are otherwise well known in the art of PNA synthesis and peptide synthesis. Labels:
Non-limiting examples of detectable moieties (labels) suitable for labeling probes or antibodies used in the practice of this invention would include a dextran conjugate, a branched nucleic acid detection system, a chromophore, a fluorophore, a spin label, a radioisotope, an enzyme, a hapten, an acridinium ester and a chemiluminescent compound. Other suitable labeling reagents and preferred methods of attachment would be recognized by those of ordinary skill in the art of PNA, peptide or nucleic acid synthesis.

Preferred haptens include 5(6)-carboxyfluorescein, 2,4-dinitrophenyl, digoxigenin, and biotin.

Preferred fluorochromes (fluorophores) include 5(6)-carboxyfluorescein (Flu), 6-((7-amino-4-methylcoumarin-3-acetyl)amino)hexanoic acid (Cou), 5(and 6)-carboxy X-rhodamine (Rox), Cyanine 2 (Cy2) Dye, Cyanine 3 (Cy3) Dye, Cyanine 3.5 (Cy3.5) Dye, Cyanine 5 (Cy5) Dye, Cyanine 5.5 (Cy5.5) Dye Cyanine 7 (Cy7) Dye, Cyanine 9 (Cy9) Dye (Cyanine dyes 2, 3, 3.5, 5 and 5.5 are available as NHS esters from Amersham, Arlington Heights, IL), JOE, Tamara or the Alexa dye series (Molecular Probes, Eugene, OR).

Preferred enzymes include polymerases (e.g. Taq polymerase, Klenow PNA polymerase, T7 DNA polymerase, Sequenase, DNA polymerase 1 and phi29 polymerase), alkaline phosphatase (AP), horseradish peroxidase (HRP) and most preferably, soy bean peroxidase (SBP).

### Detectable And Independently Detectable Moieties/Multiplex Analysis:

In this invention, a multiplex hybridization assay is performed. In a multiplex assay, numerous conditions of interest are simultaneously examined. Multiplex analysis relies on the ability to sort sample components or the data associated therewith, during or after the assay is completed. In preferred embodiments of the invention, one or more distinct independently detectable moieties are used to label two or more different probes used in an assay. The ability to differentiate between and/or quantitate each of the independently detectable moieties provides the means to multiplex a hybridization assay because the data that correlates with the hybridization of each of the distinctly (independently) labeled probe to a particular target sequence can be correlated with the presence, absence or quantity of each organism sought to be detected in the sample. Consequently, the multiplex assays of this invention may be used to simultaneously detect the presence, absence or quantity of two or more organisms in the same sample and in the same assay.

### Unlabeled Probes:

The probes that are used for the practice of this invention need not be labeled with a detectable moiety to be operable within the methods of this invention. It is possible to detect the probe/target sequence complex formed by hybridization of the probing nucleobase sequence of the probe to the target sequence using an antibody raised to bind to the probe/target sequence complex. As a non-limiting example, a PNA/nucleic acid complex could be detected using an antibody that specifically interacts with the complex, under suitable antibody binding conditions. Suitable antibodies to PNA/nucleic acid complexes and methods for their preparation and use are described in WIPO Patent Application WO95/17430 as well as US 5,612,458. Similarly, antibodies to DNA/DNA hybrids are well known in the art and can be made and used as described in US 5,200,313.

Given the discussion set forth above, it becomes apparent that the detectable and independently detectable probes discussed herein may be selected to be of different types so that their hybrids can be distinguished (detectable probe/target sequence to be distinguished from the independently detectable probe/target sequence). However, distinguishing between these hybrids depends on which type (e.g. DNA, RNA or PNA) of probe is chosen for each of the detectable and independently detectable probes since specific independently detectable antibodies can be used to bind to the differing probe/target sequence compositions.

For example, all detectable probes can be PNA and all independently detectable probes can be DNA or RNA. All the PNA/target sequence complexes will thus be detectable using a detectable antibody to the PNA/target sequence complex but the DNA or RNA/target sequence complex will not be detectable using that antibody. However, all the DNA or RNA/target sequence complexes are independently detectable using an independently detectable antibody to said unique DNA or RNA/target sequence complex. Importantly, the PNA/target sequence will not be detectable using said independently detectable antibody. Hence, it becomes clear that the probe of this invention need not be directly labeled with detectable moieties to be operable within the methods (including multiplex methods) described herein so long as probes can be made to be detectable and independently detectable.

### Self-Indicating "Beacon" Probes:

The labels attached to the "Beacon" probes comprise a set (hereinafter "Beacon Set(s)") of energy transfer moieties comprising at least one energy transfer donor and at least one energy transfer acceptor moiety. Typically, the Beacon Set will include a single donor moiety and a single acceptor moiety. Nevertheless, a Beacon Set may contain more than one donor moiety and/or more than one acceptor moiety. The donor and acceptor moieties operate such that one or more acceptor moieties accepts energy transferred from the one or more donor moieties or otherwise quench signal from the donor moiety or moieties. Though the previously listed fluorophores (with suitable spectral properties) might also operate as energy transfer acceptors, preferably, the acceptor moiety is a quencher moiety. Preferably, the quencher moiety is a non-fluorescent aromatic or heteroaromatic moiety. The preferred quencher moiety is 4-((-4-(dimethylamino)phenyl)azo) benzoic acid (dabcyl).

Transfer of energy between donor and acceptor moieties of a "Beacon" probe may occur through collision of the closely associated moieties of a Beacon Set or through a non radiative process such as fluorescence resonance energy transfer (FRET). For FRET to occur, transfer of energy between donor and acceptor moieties of a Beacon Set requires that the moieties be close in space and that the emission spectrum of a donor(s) have substantial overlap with the absorption spectrum of the acceptor(s) (See: Yaron et al. Analytical Biochemistry, 95: 228-235 (1979) and particularly page 232, col. 1 through page 234, col. 1). Alternatively, collision mediated (radiationless) energy transfer may occur between very closely associated donor and acceptor moieties whether or not the emission spectrum of a donor moiety(ies) has a substantial overlap with the absorption spectrum of the acceptor moiety(ies) (See: Yaron et al., Analytical Biochemistry, 95: 228-235 (1979) and particularly page 229, cowl. 1 through page 232, col. 1). This process is referred to as intramolecular collision since it is believed that quenching is caused by the direct contact of the donor and acceptor moieties (See: Yaron et al.).

### (i) Linear Beacons:

In a preferred embodiment, the self-indicating "Beacon" probe is a Linear Beacon as more fully described in co-pending and commonly owned patent application USSN 09/179,162, entitled: "Methods, Kits And Compositions Pertaining To Linear Beacons".

### (ii) PNA Molecular Beacons:

In a preferred embodiment, the self-indicating "Beacon" probe is a PNA Molecular Beacon as more fully described in co-pending patent application: USSN 09/179,298, entitled: "Methods, Kits And Compositions Pertaining To PNA Molecular Beacons".

### (iii) DNA Molecular Beacons:

In a preferred embodiment, the self indicating "Beacon" probe is a nucleic acid molecular beacon as more fully described in US 5,925,517, entitled: "Detectably Labeled Dual Conformation Oligonucleotide Probes, Assays and Kits".

### Detecting Energy Transfer:

Hybrid formation of a self-indicating "Beacon" probe with a target sequence can be monitored by measuring at least one physical property of at least one member of the Beacon Set that is detectably different when the hybridization complex is formed as compared with when the "Beacon" probe exists in the absence of target sequence. We refer to this phenomenon as the self-indicating property of "Beacon" probes. This change in detectable signal results from the change in efficiency of energy transfer between the donor and acceptor caused by hybridization of the "Beacon" probe to the target sequence. Preferably, the means of detection will involve measuring fluorescence of a donor or acceptor fluorophore of a Beacon Set. Most preferably, the Beacon Set will comprise at least one donor fluorophore and at least one acceptor quencher such that the fluorescence of the donor fluorophore is will be used to detect, identify or quantitate hybridization of the probe to the target sequence.

### Other Self-Indicating Probes:

In another embodiment, the self-indicating probes used in this invention are of the type described in WIPO patent application WO97/45539. The self-indicating probes described in WO97/45539 differ as compared with "Beacon" probes primarily in that the reporter must interact with the nucleic acid to produce signal. Preferably, the probes of WO97/45539,as used in this invention, are appropriately labeled peptide nucleic acids that produce detectable signal upon hybridization to the target sequence.

### Spacer/Linker Moieties:

Generally, spacers are used to minimize the adverse effects that bulky labeling reagents might have on hybridization properties of probes. Linkers typically induce flexibility and randomness into the probe or otherwise link two or more nucleobase sequences of a probe. Preferred spacer/linker moieties for the nucleobase polymers of this invention consist of one or more aminoalkyl carboxylic acids (e.g. aminocaproic acid) the side chain of an amino acid (e.g. the side chain of lysine or ornithine) natural amino acids (e.g. glycine), aminooxyalkylacids (e.g. 8-amino-3,6-dioxaoctanoic acid), alkyl diacids (e.g. succinic acid), alkyloxy diacids (e.g. diglycolic acid) or alkyldiamines (e.g. 1,8-diamino-3,6-dioxaoctane). Spacer/linker moieties may also incidentally or intentionally be constructed to improve the water solubility of the probe (For example see: Gildea et al., Tett. Lett. 39: 7255-7258 (1998)). Preferably, a spacer/linker moiety comprises one or more linked compounds having the formula: -Y-(Oₘ-(CW₂)ₙ)ₒ-Z-. The group Y is selected from the group consisting of: a single bond, -(CW₂)ₚ-,-C(O)(CW₂)ₚ-, -C(S)(CW₂)ₚ- and -S(O₂)(CW₂)ₚ. The group Z has the formula NH, NR², S or O. Each W is independently H, R², -OR², F, Cl, Br or I; wherein, each R² is independently selected from the group consisting of: -CX_{3,} -CX₂CX_{3,} -CX₂ CX₂ CX₃, - CX₂(CX₃)₂ and-C(CX₃)₃. Each X is independently H, F, Cl, Br or I. Each m is independently 0 or 1. Each n, o and p are independently integers from 0 to 10.

### Hybridization Conditions/Stringency:

Those of ordinary skill in the art of nucleic acid hybridization will recognize that factors commonly used to impose or control stringency of hybridization include formamide concentration (or other chemical denaturant reagent), salt concentration (i.e., ionic strength), hybridization temperature, detergent concentration, pH and the presence or absence of chaotropes. Optimal stringency for a probe/target sequence combination is often found by the well known technique of fixing several of the aforementioned stringency factors and then determining the effect of varying a single stringency factor. The same stringency factors can be modulated to thereby control the stringency of hybridization of a PNA to a nucleic acid, except that the hybridization of a PNA is fairly independent of ionic strength. Optimal stringency for an assay may be experimentally determined by examination of each stringency factor until the desired degree of discrimination is achieved.

### Suitable Hybridization Conditions:

Generally, the more closely related the background causing nucleic acid contaminates are to the target sequence, the more carefully stringency must be controlled. Blocking probes may also be used as a means to improve discrimination beyond the limits possible by mere optimization of stringency factors. Suitable hybridization conditions will thus comprise conditions under which the desired degree of discrimination is achieved such that an assay generates an accurate (within the tolerance desired for the assay) and reproducible result. Aided by no more than routine experimentation and the disclosure provided herein, those of skill in the art will easily be able to determine suitable hybridization conditions for performing assays utilizing the methods and compositions described herein. Suitable *in-situ* hybridization conditions comprise conditions suitable for performing an *in-situ* hybridization procedure. Thus, suitable *in-situ* hybridization conditions will become apparent to those of skill in the art using the disclosure provided herein; with or without additional routine experimentation.

### Blocking Probes:

Blocking probes are nucleic acid or non-nucleic acid probes that can be used to suppress the binding of the probing nucleobase sequence of the probing polymer to a non-target sequence. Preferred blocking probes are PNA probes (See: Coull et al., US 6,110,676). Typically blocking probes are closely related to the probing nucleobase sequence and preferably they comprise one or more single point mutations as compared with the probe sought to be detected in the assay. It is believed that blocking probes operate by hybridization to the non-target sequence to thereby form a more thermodynamically stable complex than is formed by hybridization between the probing nucleobase sequence and the non-target sequence. Formation of the more stable and preferred complex blocks formation of the less stable non-preferred complex between the probing nucleobase sequence and the non target sequence. Thus, blocking probes can be used with the methods of this invention to suppress the binding of the nucleic acid or non-nucleic acid probe to a non-target sequence that might be present and interfere with the performance of the assay. Blocking probes are particularly advantageous in single point mutation discrimination.

### Probing Nucleobase Sequence:

The probing nucleobase sequence of the probes used in this invention is the specific sequence recognition portion of the construct. Therefore, the probing nucleobase sequence is a nucleobase sequence designed to hybridize to a specific target sequence wherein the presence, absence or amount of the target sequence can be used to directly or indirectly detect the presence, absence or number of organisms of interest in a sample. Consequently, with due consideration to the requirements of a probe for the assay format chosen, the length and sequence composition of the probing nucleobase sequence of the probe will generally be chosen such that a stable complex is formed with the target sequence under suitable hybridization conditions.

The preferred probing nucleobase sequence of me probes of this invention that are suitable for detecting Salmonella bacteria comprise a nucleobase sequence selected from the group consisting of: GTG-TTA-AAG-TGA-ACC (Seq. ID No. 1), AGC-CTT-GAT-TTT-CCG (Seq. ID No. 2) or ACC-TAC-GTG-TCA-GCG (Seq. ID No. 3), and the complement thereto. PNA probes selected to have these nucleobase sequences have been shown by Applicant to be highly specific for Salmonella while not exhibiting a substantial cross reaction to bacteria of the closely related Citrobacter genus, except for Seq. ID No 1 which exhibits some weak cross with certain strains of Citrobacter bacteria.

This invention contemplates that variations in these identified probing nucleobase sequences shall also provide probes that are suitable for the specific detection of Salmonella while still discriminating Citrobacter. Common variations include, deletions, insertions and frame shifts. Additionally, a shorter probing nucleobase sequence can be generated by truncation of the above identified sequence. Alternatively, if the probing nucleobase sequence is nucleic acid (e.g. DNA or RNA), typically the probing nucleobase sequence will be longer than 15 nucleobases and designed by extending the above identified nucleobase sequences using the readily available rRNA sequence information to determine the one or more desired additional flanking nucleobase(s). In general however, the probing nucleobase sequence is preferably 7-25 subunits in length and most preferably 7-15 subunits in length.

A probe for use in this invention will generally have a probing nucleobase sequence that is exactly complementary to the target sequence. Alternatively, a substantially complementary probing nucleobase sequence might be used since it has been demonstrated that greater sequence discrimination can be obtained when utilizing probes wherein there exists one or more point mutations (base mismatch) between the probe and the target sequence (See: Guo et al., Nature Biotechnology 15:331-335 (1997)). Consequently, the probing nucleobase sequence may be only 90% homologous to the probing nucleobase sequences identified above, or their complements. Complements of the probing nucleobase sequence are included since it is possible to generate a suitable probe if the target sequence to be detected has been amplified or copied to thereby generate the complement to the identified target sequence of the target molecule; such as a cDNA or cRNA.

### Probe Complexes:

In still another embodiment, two probes are designed to, in the aggregate, produce a probing nucleobase sequence that hybridizes to the target sequence sought to be detected and thereby generates a detectable signal whereby the nucleobase sequence of each probe comprises half or approximately half of the complete complement to the target sequence. As a non-limiting example, the nucleobase sequences of the two probes might be designed using the assay as described in European Patent Application 849,363, entitled "Method of identifying a nucleic add using triple helix formation of adjacently annealed probes" by H. Orum et al. (See: EPA 849,363). Similar compositions comprising a PNA probe triplex have been described in copending and commonly owned application USSN 09/302,238. Using this methodology, the probes that hybridize to the target sequence may or may not be labeled since it is the probe complex formed by the annealing of the adjacent probes that is directly detected and not the probes that directly bind to the target sequence.

### III: Preferred Embodiments of the Invention:

### a. PNA Probes:

In one embodiment, this invention uses probes suitable for the detection of bacteria of the Salmonella genus that have the advantage that they do not substantially cross react with bacteria of the closely related Citrobacter genus. General characteristics (e.g. length, labels, nucleobase sequences, linkers etc.) of probes suitable for the detection, identification and/or quantitation of Salmonella bacteria have been previously described herein The preferred probing nucleobase sequence is selected from the group consisting of: GTG-TTA-AAG-TGA-ACC (Seq. ID No. 1), AGC-CTT-GAT-TTT-CCG (Seq. ID No. 2) or ACC-TAC-GTG-TCA-GCG (Seq. ID No. 3). Preferably, the probes of the set are peptide nucleic acids and most preferably, the probing nucleobase sequence consists of only PNA subunits.

Each of the preferred probing nucleobase sequences set forth above are chosen because they exhibit a particular specificity for bacteria of the Salmonella genus. They also exhibit a particular cross reactivity. Seq. ID No. 1 is designed to detect approximately one half of all known Salmonella bacteria. Seq. ID No.1 1 exhibits reactivity with only a few species of Citrobacter bacteria.

Seq. ID No. 2 is chosen because it detects many of the Salmonella bacteria that are not detectable using Seq. ID No.1. Thus, the combination of Seq. ID Nos. 1 and 2, in the aggregate, detect most known Salmonella bacteria. However, certain of known Salmonella species, such as Salmonella salmenae is not detectable using the combination of Seq. ID Nos. 1 and 2.

Seq. ID No. 2, however does exhibit cross reactivity with E. coli bacteria (one of the USP bacteria). Because E. coli is so common, this sequence is likely to always produce some false positive result unless the result is somehow, corrected for the E. coli content of the sample. Nevertheless, the present invention makes it possible to correct for this cross reactivity as described below under the section entitled: "Corrective Detection Methods".

Seq. ID No. 3 is the most specific of the above identified probing nucleobase sequences. Seq. ID. No 3 is believed to not cross react with either of Citrobacter bacteria or E. coli bacteria. Seq. ID. No 3 is also believed to detect known Salmonella enterica bacteria including Salmonella salmenae. Seq. ID No. 3 does however, cross react with the very rare Yersinia enterocolitica. However, because Yersinia enterocolitica is not that common, the incidence of obtaining a false positive result is very low. Nevertheless, it is again possible to correct for this cross reactivity with the method of the present invention as described below under the section entitled: "Corrective Detection Methods".

The probes for use in this invention may comprise only a probing nucleobase sequence or may comprise additional moieties. Non-limiting examples of additional moieties include detectable moieties (labels), linkers, spacers, natural or non-natural amino acids, or other subunits of PNA, DNA or RNA. Additional moieties may be functional or non-functional in an assay. Generally however, additional moieties will be selected to be functional within the design of the assay in which the probe is to be used. The probes for use in this invention may be unlabeled but the preferred probes of this invention are labeled with one or more detectable moieties. In a more preferred embodiment, one or more probes are labeled with two or more independently detectable moieties. Preferred independently detectable moieties are independently detectable fluorophores.

In preferred embodiments, the probes are used in *in-situ* hybridization (ISH) and fluorescence *in-situ* hybridization (FISH) assays. Excess probe used in a ISH or FISH assay typically must be removed so that the detectable moiety of the specifically bound probe can be detected above the background signal that results from still present but unhybridized probe. Generally, the excess probe is washed away after the sample has been incubated with probe for a period of time. However, the use of "beacon" probes or other self indicating probes is a preferred embodiment of this invention, since there is no requirement that excess self-indicating probe be completely removed (washed away) from the sample since it generates little or no detectable background.

The probes may optionally be immobilized to a surface, including immobilization in an array of probes. Probes can be immobilized to the surface using the well known process of UV-crossling. More preferably, the probe is synthesized on the surface in a manner suitable for deprotection but not cleavage from the synthesis support (See: Weiler, J. et al, Hybridization based DNA screening on peptide nucleic acid (PNA) oligomer arrays., Nucl. Acids Res., 25;14:2792-2799 (July 1997)). In still another embodiment, one or more probes are covalently linked to a surface by the reaction of a suitable functional group on the probe with a functional group of the surface (See: Lester, A. et al, "PNA Array Technology": Presented at Biochip Technologies Conference in Annapolis (October 1997)). This method is most preferred since the probes on the surface will typically be highly purified and attached using a defined chemistry, thereby minimizing or eliminating non-specific interactions.

Methods for the chemical attachment of probes to surfaces generally involve the reaction of a nucleophilic group, (e.g. an amine or thiol) of the probe to be immobilized, with an electrophilic group on the support to be modified. Alternatively, the nucleophile can be present on the support and the electrophile (e.g. activated carboxylic acid) present on the probe. Because native PNA possesses an amino terminus, a PNA will not necessarily require modification to thereby immobilize it to a surface (See: Lester et al., Poster entitled "PNA Array Technology").

Conditions suitable for the immobilization of a probe to a surface will generally be similar to those conditions suitable for the labeling of the polymer. The immobilization reaction is essentially the equivalent of labeling whereby the label is substituted with the surface to which the polymer is to be linked.

Numerous types of surfaces derivatized with amino groups, carboxylic acid groups, isocyantes, isothiocyanates and malimide groups are commercially available. The support may be flat, round (e.g. beaded) or of any other useful geometric shape. Non-limiting examples of suitable surfaces include membranes, glass, controlled pore glass, polystyrene particles (beads), silica and gold nanoparticles.

### b. Probe Sets:

In another embodiment, this invention uses probe sets suitable for detecting, identifying and/or enumerating bacteria of the Salmonella genus that have the advantage that they do not substantially cross react with bacteria of the closely related Citrobacter genus. The general and preferred characteristics of the probes that are suitable for the detection, identification and/or enumeration of bacteria of the Salmonella genus been previously described herein. The probes sets preferably comprise at least one probe having a probing nucleobase sequence that is at least ninety percent homologous to the nucleobase sequence: GTG-TTA-AAG-TGA-ACC (Seq. ID No. 1), AGC-CTT-GAT-TTT-CCG (Seq. ID No. 2), ACC-TAC-GTG-TCA-GCG (Seq. ID No. 3) and the complements thereof. Preferably, the probes of the set are peptide nucleic acids and most preferably, the probing nucleobase sequence consists of only PNA subunits.

The probe sets may comprise mixtures of PNA probes and nucleic acid probes, provided however that a set comprises at least one probe suitable for detecting, identifying and/or enumerating bacteria of the Salimonella genus, as described above. In preferred embodiments, some of the probes of the set are blocking probes composed of PNA or nucleic acid but preferably the blocking probes are PNA. In other preferred embodiments, the probes of the set are support bound. Most preferably, all of the probes of a set are PNA.

In preferred embodiments, the probe sets comprise two or preferably all three of the preferred probing nucleobase sequences identified above as Seq. ID Nos. 1-3 so as to enable the detection of all Salmonella bacteria that may be present in a sample. For example, Seq. ID Nos. 1 and 2 are typically combined so that most of the Salmonella bacteria are detectable since each individual Seq. ID No. only detects approximately half of the known Salmonella species. Adding Seq. ID No. 3 to the mixture increases signal intensity and also will allow for the detection of rare Salmonella bacteria that might not be detectable with even Seq. ID Nos. 1 and 2. Therefore, it is the combination of all of Seq. ID Nos. 1-3 that is preferred for the general detection of Salmonella bacteria.

The probe set may comprise the two or all three of Seq. ID Nos. 1-3 despite the expected presence of bacteria with which each is known to cross react, provided that the result can be corrected for the presence of these bacteria via the method of the invention, as described below under the section entitled: "Corrective Detection Methods". In this way, the additional probes of the set correct for the deficiencies of specificity of the identified Seq. ID Nos. 1-3.

Arrays are surfaces to which two or more probes have been immobilized each at a specified unique location. Typically, the probing nucleobase sequence of the immobilized probe is judiciously chosen to interrogate a sample. Because the location and composition of each immobilized probe is known, arrays are generally useful for the simultaneously detection, identification or quantitation of two or more target sequences that may be present in the sample. Therefore, in another embodiment, two or more probes of the set are each immobilized at different identifiable locations on the array so that upon hybridization of the probe to the target sequence, the presence of the hybrid at the location on the array can be used to identify, detect and/or enumerate the target sequence of interest.

Because the probes of this invention can be labeled with one or more independently detectable moieties, it is possible to design probe sets wherein each probe of a set is independently detectable. Fluorophores that have sufficiently different excitation and emission spectra are often used as independently detectable moieties. Exemplary independently detectable fluorophores are derivatives of coumarin, fluorescein and rhodamine.

### c. Corrective Detection Methods:

As noted above, this invention is directed to a method for specifically detecting, identifying and/or quantitating (enumerating) bacteria of the Salmonella genus in a sample, among other detectable organisms with which probes for the Salmonella bacteria will cross react. This is also referred to herein as a corrective detection method.

The method comprises contacting the sample, under suitable hybridization conditions, with one or more detectable probes suitable for detecting, identifying and/or quantitating bacteria of the Salmonella genus as well as with one or more independently detectable probes that are specific for one or more other detectable organisms with which the detectable probes for the bacteria of interest will cross react, wherein the independently detectable probes do not themselves cross react with the Salmonella bacteria. Hybridization, under suitable hybridization conditions, of the probing nucleobase sequence of the detectable and independently detectable probes to their target sequences is then determined by directly or indirectly detecting the probe/target sequence complexes in the sample. The presence, absence and/or number of organisms that are stained with both the detectable probes and the independently detectable probes is then determined so that those bacteria that are stained with only the detectable probe or probes can be specifically determined for purposes of specific detection, identification and/ox quantitation of the Salmonella bacteria in the sample. In this manner, there is a correction made for the non-specific character of the probe or probes chosen for specific detection of the Salmonella bacteria.

Because this method can be used to specifically determine the organisms that exhibit cross reactivity with the Salmonella bacteria, this invention facilitates the use of probes of less than optimal specificity in an assay for specifically determining Salmonella bacteria in a sample of interest. Even if the probe sequence or sequences chosen are not, or cannot be, made specific for the Salmonella bacteria sought to be determined, it is possible to subtract out the organisms that exhibit cross reactivity and thereby achieve the desired specificity. The only requirement is that the independently detectable probe or probes chosen to detect the troublesome organism or organisms do not themselves exhibit cross reactivity with the Salmonella bacteria of interest.

Example 9 of this specification is a representative Example of such a method. In this Example, a microscope, CCD camera and appropriate software are used to analyze the stained bacteria in the sample. The sample analyzed comprises the following bacteria: S. choleraesuis, S. arizonzae, P. aeruginosa, B. cepatia, S. aureus, and E. coli. The green probe used in the assay is the PNA, Flu-2, comprising Seq. ID No. 2 (See: Table 1). Since the probing nucleobase sequence of the probe is Seq. ID No. 2, Flu-2 is expected to cross react with E. coli. The red probe used in the assay is the PNA, Cy3-1 (See: Table 1). As noted in Table 1, this probe is specific for E. coli.

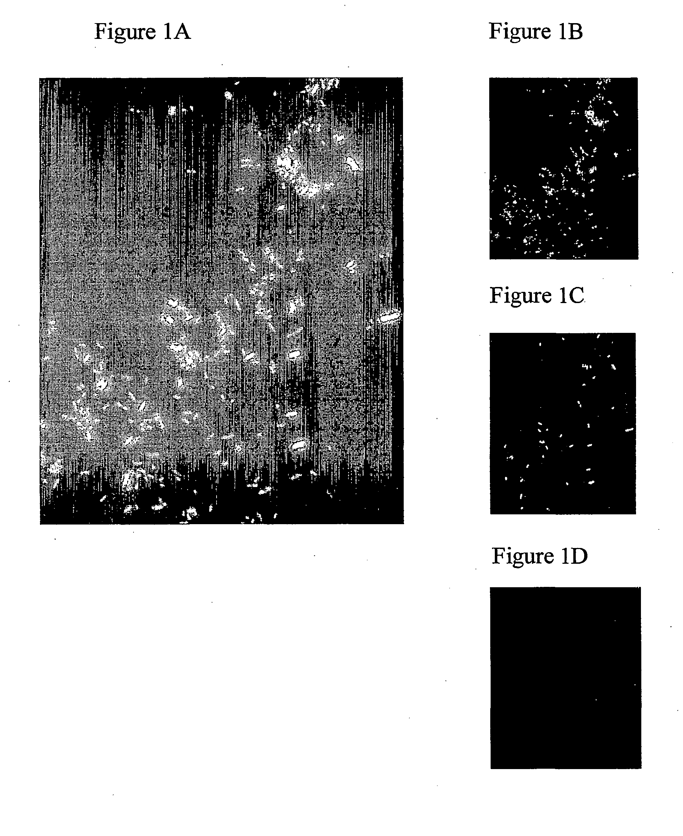

With reference to Figures 1A-1D, an embodiment of the method is discussed. Figures 1B,1C and 1D are the individual digital images obtained with a microscope using each of the blue, green and red filters, respectively. These three images are then digitally combined to formulate the composite digital image seen in Figure 1A. The blue image (Figure 1B) shows all bacteria present in the sample that are stained with the general blue dye, DAPI. The green image (Figure 1C) shows all bacteria in the sample with which the PNA, Flu-2, reacts to thereby generate a detectable green signal. Since Salmonella and E. coli bacteria are present in the sample, these bacteria are both stained green. The red image (Figure 1D) shows only those bacteria that react with the Cy3-1 probe. Because the PNA probe Cy3-1 is specific, only E. coli bacteria should be stained red.

With reference to the composite digital image presented in Figure 1A, all bacteria in the sample are seen as either yellow, green or blue. Yellow seen in the composite image is a computer generated pseudocolor that is assigned to those areas of the image that are both green and red. Thus, the yellow stained bacteria are the E. coli bacteria since only E. coli bacteria are expected to be stained with both the green (Flu-2) and red (Cy3-1) probes. The absence of bacteria that are only red indicates that the Cy3-1 probe is specific for E. coli and does not generally cross react with other bacteria in the sample.

Having corrected for the E. coli, the remaining green bacteria are therefore the Salmonella bacteria. As can be seen, most bacteria are blue. This indicates that the Flu-2 probe is also specific in that it does not generally stain all bacteria. Thus, most of the bacteria in the field of the microscope are not E. coli or Salmonella. It is also noted that that observed cell morphology is consistent with the identifications made based on cell color.

Although the methodology discussed above is based on the manual analysis of computer generated digital images, the process could be automated using appropriate instrumentation and software in combination with a slide scanner or flow cytometer. Most preferably the instrumentation and software is designed to automatically perform the inventive corrective method described herein.

Although Example 9 demonstrates only a correction for E. coli, it is not a limitation of the corrective method that only one type of cross reaction be corrected per assay. Because the probes and probe sets can be used in a multiplex format, all cross reacting organisms can potentially be corrected. For example, a Cy3 stained probe designed to specifically detect Yersinia enterocolitica, a Cy3 probe designed to specifically detect Yersinia pseudotuberculosis and another Cy-3 labeled probe designed to specifically detect , Citrobacter bacteria could be added to the Cy3-1 probe described herein and used to completely correct for organisms with which a mixture of fluorescein labeled probes (See: Table 1, probes Flu-1, Flu-2 and Flu-3 as examples) comprising Seq. ID Nos. 1-3 cross react. In this manner, the assay would be highly specific for Salmonella bacteria wherein a correction is made for all organisms known to cross react with the detectable probes.

As previously discussed, the probes need not be labeled with a detectable moiety to be operable within the corrective methods of this invention since it is possible to detect the probe/target sequence complex without using a labeled probe or labeled target sequence. For example, the detectable and independently detectable probes can be of different types whereby the detectable probe/target sequence complex is distinguishable as compared with the independently detectable probe/target sequence complex.

In preferred embodiments, the probes used in the corrective method described above are self-indicating "beacon" probes or other self-indicating probes. When self-indicating probes are used, the method can be a real-time assay since excess probe does not substantially interfere with the analysis of hybridization and therefore does not need to be separated away.

### d. Exemplary Applications For Using The Invention:

The methods of this invention are particularly useful for the rapid, sensitive and reliable detection of Salmonella bacteria in food, beverages, water, pharmaceutical products, personal care products, dairy products or environmental samples. The analysis of preferred beverages include soda, bottled water, fruit juice, beer, wine or liquor products. Suitable methods will be particularly useful for the analysis of raw materials, equipment, products or processes used to manufacture or store food, beverages, water, pharmaceutical products, personal care products, dairy products or environmental samples.

The methods of this invention are also particularly useful for the rapid, sensitive and reliable detection of Salmonella bacteria (pathogens) in clinical environments. Suitable methods will be particularly useful for the analysis of clinical specimens, equipment, fixtures or products used to treat humans or animals.

### Brief Description of the Drawings

Figure 1A is a composite digital image obtained with each of the blue, green and red filters of a CCD camera attached to a microscope wherein yellow is a pseudocolor assignment that identifies bacteria visible in both the green and red images.
Figures 1B,1C and 1D are the individual digital images obtained with the microscope using each of the blue, green and red filters, respectively, that are used to formulate the composite digital image seen in Figure 1A.

### Modes For Carrying Out The Invention

This invention is now illustrated by the following examples that are not intended to be limiting in any way.

### Example 1: Synthesis of bis-(2-methoxyethyl)amidyl-diglycolic acid

To 500 mmol of diglycolic anhydride stirring in 800 mL of dichloromethane (DCM) was added dropwise,1.1 mole of bis(2-methoxyethyl)amine (Aldrich Chemical). The reaction was allowed to stir for 2 hours and then 280 mL of 6N HCl was added dropwise. The contents were then transferred to a separatory funnel and allowed to separate. The DCM layer was removed and the aqueous layer extracted with 100 mL of DCM. The combined DCM layers were then extracted with 100 mL of 10% aqueous citric acid. The DCM layer was then separated, dried (Na₂SO₄), filtered and evaporated to yield 73.8 g (296 mmole; 59 % yield). A kugelrorh was then used to remove traces of solvent (product was heated to 60 °C at approximately 180 µM Hg, but was not distilled).

### Example 2: Synthesis of N-[N"-Fmoc-(2"-aminoethyl)-N-[N,N'-(2-methoxyethyl)amidyl-diglycolyl]glycine ("Fmoc-"E" aeg-OH")

To 60 mmol of Fmoc-aeg-OH (PE Biosystems, Foster City, CA.) was added 360 mL of MilliQ water, 180 mL of acetone, 120 mmol of NaHCO₃ and 60 mmol of K₂CO₃. This solution was allowed to stir until all the Fmoc-aeg-OH had dissolved (approx. 2 hr.) and then the solution prepared, as described below, was added.

To 70 mmol of bis-(2-methoxyethyl)amidyl-diglycolic acid was added 120 mL of anhydrous acetonitrile (Fluka Chemical), 240 mmol of N-methylmorpholine (NMM; Fluka Chemical) and 75 mmol of trimethylacetyl chloride (Aldrich Chemical). The solution was allowed to stir at room temperature for 30 minutes and then added dropwise to the solution of Fmoc-aeg-OH that was prepared as described above.

After the combined solutions stirred for 1 hr and tlc analysis indicated complete reaction, 6N HCl was added to the reaction until the pH was less than 2 by paper. The organic solvent was then removed by vacuum evaporation. The remaining aqueous solution was then transferred to a separatory funnel and extracted 2 x with 250 mL of ethyl acetate. The combined ethyl acetate layers were dried (Na₂SO₄), filtered and evaporated to yield 41.5g of an oil.

This crude product was purified by column chromatography using a reversed phase stationary phase (C18) and a gradient of aqueous acetonitrile to elute the product and remove the pivalic acid. Though not visible by tlc, the elution of the pivalic acid was monitored by smell. The pivalic acid was almost completely eluted from the column prior to elution of the product. Elution of the product was monitored by tlc. Yield 26.8g (47 mmol; 78%). An "E" modification of a PNA or polyamide has the formula:

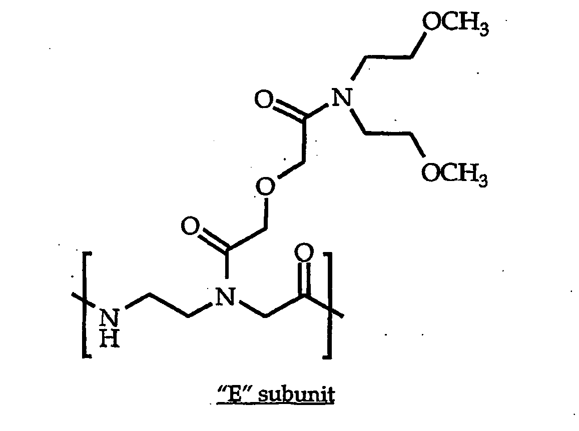

### Example 3: Synthesis of PNAs

Unless, otherwise stated, PNAs were synthesized using commercially available reagents and instrumentation obtained from PE Biosystems, Foster City, CA. USA. PNAs possessing C-terminal "E" subunit modifications were prepared by performing the synthesis using prederivatized synthesis support or by performing the synthesis using the Fmoc-"E"aeg-OH (prepared as described above) monomer and the standard synthesis cycle. PNAs possessing N-terminal terminal "E" subunit modifications were prepared by performing the synthesis using the Fmoc-"E"aeg-OH (prepared as described above) monomer and the standard synthesis cycle.

### Example 4: Preferred Method For Removal Of The Fmoc Protecting Group

The synthesis support was treated with a solution of 25% piperidine in DMF for 10-15 minutes at room temperature. After treatment, the synthesis support was washed and dried under high vacuum. The support can then be treated with labeling reagent.

### Example 5: Preferred Procedure For Labeling Of Support Bound PNA With 5(6)carboxyfluorescein

After proper reaction with linkers and removal of the terminal amine protecting group, the resin was treated with 250 µL of a solution containing 0.5M 5(6)carboxyfluorescein, 0.5M N,N'-diisopropylcarbodiimide, 0,5M 1-hydroxy-7-azabenzotriazole (HOAt) in DMF (See: Weber et al., Bioorganic & Medicinal Chemistry Letters, 8: 597-600 (1998). After treatment, the synthesis support was washed and dried under high vacuum. The PNA oligomer was then cleaved, deprotected and purified as described below.

### Example 6: General Procedure For Cleavage, Deprotection and Purification PNA Oligomers Prepared:

| **Table 1** | | |
|---|---|---|
| **Probe ID** | **Target Organism** | **PNA Probe Sequence** |
| Flu-1 | Salmonella bacteria | Flu-OEE-GTG-TTA-AAG-TGA-ACC-EE-NH₂ |
| Flu-2 | Salmonella bacteria | Flu-OEE-AGC-CTT-GAT-TTT-CCG-EE-NH₂ |
| Flu-3 | Salmonella bacteria | Flu-OEE-ACC-TAC-GTG-TCA-GCG-EE-NH₂ |
| Cy3-1 | E. coli bacteria | Cy3-OEE-TCA-ATG-AGC-AAA-GGT-EE-NH₂ |

All PNA sequences are written from the amine (N-) terminus to the carboxyl (C-) terminus. Flu = 5(6)-carboxyfluorescein; E is defined above; and O = 8-amino-3,6-dioxaoctanoic acid

The synthesis support (Fmoc-PAL-PEG/PS; P/N GEN913384) was then removed from the synthesis cartridge, transferred to a Ultrafree spin cartridge (Millipore Corp., P/N SE3P230J3) and treated with a solution of TFA/m-cresol according to manufactures instructions. The solution was spun through the support bed and again the support was treated with a solution of TFA/m-cresol. The solution was again spun through the support bed. The combined eluents (TFA/m-cresol) were then precipitated by addition of approximately 1 mL of diethyl ether. The precipitate was pelletized by centrifugation. The pellet was then resuspended in ethyl ether and pelletized two additional times. The dried pellet was then resuspended in 20% aqueous acetonitrile (ACN) containing 0.1 % TPA (additional ACN was added as necessary to dissolve the pellet). The product was analyzed and purified using reversed phase chromatographic methods.

### Example 7: Dot Blot Assays

### RNA Preparation:

Using a Qiagen kit (P/N 75144), total RNA (including app. 80 % rRNA) was isolated from the different bacterial cells which had been grown in culture. The total concentration of isolated RNA was determined by measuring the absorption at 260 nm.

### PNA Probe Hybridization to the Membranes:

Dot blots were made on nylon membranes obtained from Gibco-BRL (P/N 14830-012). For the rRNA of each cultured bacteria, a dilution row containing at least 5 spots was made, starting with a concentration of approximately 16 µg/mL RNA for the strongest dilution and continuing with half log dilutions in diethyl pyrocarbonate (DEPC) treated water (RNase free). Prior to spotting on the membrane, each dilution stock was heated to 68°C for three minutes. The spotting produced a half log dilution series containing approximately 16, 5.1,1.6, 0.52, 0.17 ng ... (etc.) total RNA per spot. Once the spots had air dried, the membrane was UV-cross linked and then stored in a plastic bag until used.

When used, individual membranes were placed in plastic bags and pre-wet with RNase free water. The membranes were prehybridized in Hybridization Buffer (20 mM Tris-HCl, pH 9.5; 50% formamide; 1X Denhardt's Solution (USB, P/N US70468); 0.7% polyoxyethylene-sorbitan monolaurate (Tween 20, Sigma P/N P-1379); and 100 mM NaCl) for 15 minutes at 50°C. All fluorescein labeled probes were diluted in 1:1 DMF/H₂O to a concentration of approximately 300 pmole/µL and then diluted to a final concentration of 5 pmol/mL each using Hybridization Buffer. The prehybridization buffer was removed from the bags and fresh hybridization buffer containing the PNA probe(s) of interest was(were) added to the bags. The appropriate probes were used at a final concentration of 5 pmol/mL each.

Probe hybridization was performed at 50°C for 1 hour. The filters were then washed 3 times in Capso-10.0 (10 mM Capso (Sigma P/N C-1254) pH 10.0, 1 mM EDTA) containing 0.2% Tween 20 at elevated temperature.

### Visualization of the Membrane:

After the washes were completed, the membranes were treated with a blocking solution (50 mM Tris-HCl, pH 9.0; 0.5 M NaCl; and 0.5% casein). The starting temperature of the solution was 65°C, but the solution cooled as the blocking proceeded with shaking at room temperature for 15 minutes. An anti-fluorescein alkaline phosphatase conjugate (Rabbit (Fab) and-FITC/AP (DAKO A/S, P/N K0046)) was diluted 1:500 in blocking solution and the membranes were left shaking in this solution for 30 minutes at room temperature. The membranes were then washed in a washing solution (50 mM Tris-HCl, pH 9.0; 0.5 M NaCl; and 0.5% Twee-20) three times each for 5 minutes. To prepare the membranes for the detection, a final rinse was performed (10 mM Tris-HCl, pH 9.5; 10 mM NaCl; and 1 mM MgCl₂). The chemiluminescent substrate (CDP Star, Tropix Corp., P/N MS025) was diluted 1:500 in an aqueous Substrate Solution (0.1 M diethanolamine, pH 9.7; and 1 mM MgCl₂) and the membranes were immersed without shaking for 4 minutes. The membranes were then placed in a plastic bag and excess substrate was squeezed out and the bag sealed. The membranes were exposed to Fuji-RX X-ray film for between 1 and 10 minutes.

### RNA Spotted Membranes:

The results of the analysis of RNA dot blots (as described above) probed with Flu-1, Flu-2, and Flu-3, are set forth in Table 2. With reference to Table 2, probe Flu-1 is specific for *Salmonella choleraesuis.* As expected based upon available sequence information, probe Flu-2 does not recognize renamed *S. choleraesuis,* the sub-species of *Salmonella* RNA used in the dot blots, but does react *with E. coli* RNA. The probe Flu-3 is specific for *S. choleraesuis*

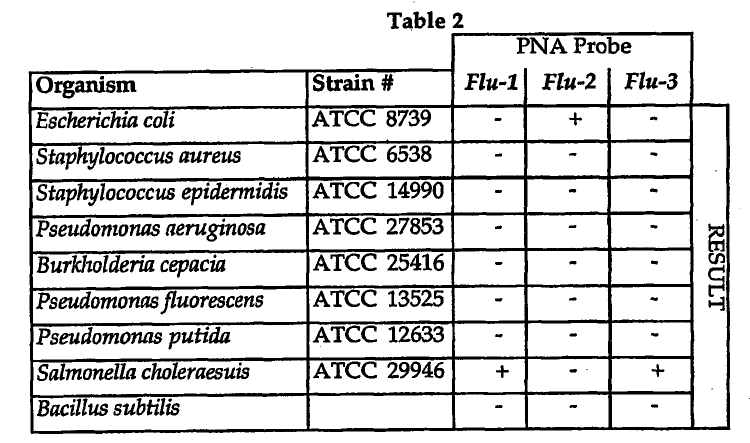

### Example 8: PNA-FISH

Individual 3 mL cultures of bacteria were grown overnight in Tryptic Soy Broth (TSB) at 30 °C. The broth was then analyzed for absorbance at 600nm and then diluted into fresh TSB until the absorbance at 600nm was approximately 0.5 OD/mL. These diluted culture stocks were then allowed to double 3-4 times before harvesting. Cells from a 20 mL culture were pelleted by centrifugation at 8000 rpm for 5 minutes, resuspended in 20 mL PBS, pelleted again and resuspended in Fixation Buffer (4 % paraformaldehyde in PBS (7 mM Na₂HPO₄; 3 mM NaH₂PO₄; 130 mM NaCl). The bacteria were incubated at room temperature for 30-60 minutes before they were pelleted again (centrifugation at 8000 R.P.M. for 5 minutes). After removal of the fixation solution, the cells were resuspended in 20 mL of sterile PBS and pelleted by centrifuging at 4000 rpm for 5 minutes. The cells were resuspended in 20 mL of 50 % aqueous ethanol. The fixed bacteria were then used after 30 minutes of incubation or optionally stored at - 20 °C for up to several weeks before being used in an experiment.

For each sample prepared, 100 µl of fixed cells in 50% aqueous ethanol was removed and centrifuged at 8000 RP.M. for 2 min. The ethanol was then remove from the sample and the pellet was resuspended in 100µl of sterile PBS and pelleted again by centrifugation at 8000 R.P.M. for 2 min.

The PBS was removed from the pellet, and the cells were resuspended in 100 µl of Hybridization Buffer (20 mM Tris-HCl, pH 9.0; 100 mM NaCl; 0.5 % SDS) that contained the appropriate probe (e.g. Flu-1 through Flu-3) at a concentration of: Flu-1, Flu-2 (90 pmol/mL), and Flu-3 (30 pmol/mL). The hybridization was performed at 55°C for 30 minutes.

The sample was then centrifuged at 8000 R.P.M. for 2 min. The hybridization buffer was removed and the cells resuspended in 500 µl sterile TE-9.0 (10 mM Tris-HCl, pH 9.0; 1 mM EDTA). The solution was allowed to stand at 55°C for 5 minutes. The sample was then centrifuged at 8000 R.P.M. for 5 minutes. The TE-9.0 was then removed from the pellet. The TE-9.0 wash was then repeated two more times.

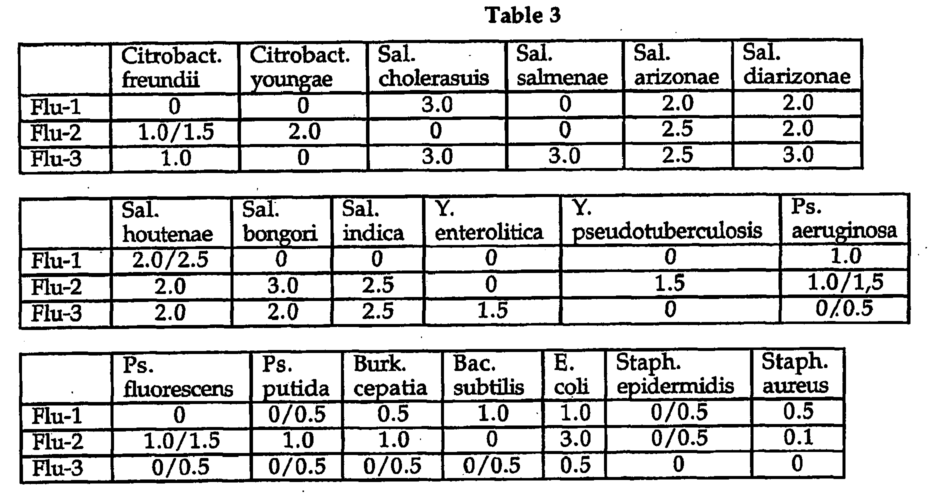

After the final wash, the cells were resuspended in 100 µl TE-9.0. An aliquot of 2 µl of this suspension of cells was placed on a glass slide, spread and allowed to dry. Next, 1-2 µl of Vectashield (Vector Laboratories, P/N H-1000) was deposited over the dried cells. A coverslip was added to the slide and its position fixed using a couple of drops of nail polish. Different bacterial strains including: *Citrobacter freundii, Citrobacter youngae, Salmonella cholerasuis, Salmonella salmenae, Salmonella arizonae, Salmonella diarizonae, Salmonella houtena, Salmonella bongori, Salmonella indica, Yersinia entercolitica, Yersinia pseudotuberculosis, Pseudomonas aeruginosa, Pseudomonas fluorescens, Pseudomonas putida, Pseudomonas cepatia, Bacillus subtilis, Escherichia coli, Staphylococcus epidermidis* and *Staphylococcus aureus,* were fixed and hybridized with each of probes Flu-1 through Flu-3 following the protocol described above. After hybridization and wash, each strain was spotted and mounted on a microscope slide (see above) and examined using a Nikon fluorescent microscope equipped with a 60 x immersion oil objective, a 10 x ocular (total enlargement is 600 fold), and an Omega Optical XF22 filter. Table 2 shows that the probes Flu-1 and Flu-2 hybridize to some of the *Salmonella* sub-species, (with different but overlapping subsets). In addition there is some cross reactivity between Flu-2 and the Citrobacter species. The Flu-3 probe recognizes each *Salmonella* sub-species, it does hybridize weakly to *Yersinia entercolitica.* For the purpose of interpreting the data in Table 3, the following interpretation should be given to the values contained therein. A value of between 0 to 1 is a negative reading wherein auto fluorescence contributes to some non-specific signal observed. A value of between 1.5 to 2 is intended to indicate a weakly positive result. A value of between 2.5 to 3 is intended to indicate a strong positive result.

### Example 9: Multiplex PNA-FISH

A mixture of *E. coli, S. aureus, P. aeruginosa, S. choleraesuis, S. arizonae,* and *P. cepatia,* fixed individually as previously described, was prepared and hybridized in a single tube with a mixture of two PNA probes, Flu-2 and Cy3-1 (See Table 1). The hybridization protocol used was the same as described in Example 8.

After hybridization, and wash, the bacteria were spotted and mounted on a microscope slide (see above). The slides were then inspected using a Nikon fluorescent microscope equipped with a 60 x immersion oil objective, a 10 x ocular (total enlargement is 600 fold) and light filters obtained from Omega Optical (XF22 (green), XF34 (red), and XF05 (blue) filter). Electronic digital images were made of each slide using a SPOT CCD-camera and software obtained from Diagnostic Instruments, Inc., Sterling Heights, MI (USA).

The digital images obtained, all covering the same section of the slide, are presented in Figure1A through 1D. An analysis of these images has previously been described in the specification.

## Claims

1. A method for specifically detecting, identifying and/or quantitating bacteria of the Salmonella genus among other detectable organisms with which a probe for the Salmonella bacteria will cross react; said method comprising:
(a) contacting the sample, under suitable hybridisation conditions, with one or more detectable probes suitable for detecting, identifying and/or quantitating bacteria of the Salmonella genus;
(b) contacting the sample, under suitable hybridisation conditions, with one or more independently detectable probes that are specific for one or more other detectable organisms with which the detectable probe(s) for the Salmonella bacteria will cross react, wherein the independently detectable probe(s) do not themselves cross react with the Salmonella bacteria;
(c) determining hybridisation of the probing nucleobase sequence of the detectable and independently detectable probes to their target nucleic acid sequences by detecting the probe/target sequence complexes in the sample; and
(d) determining the presence, absence and/or number of organisms that are stained with both the detectable probe(s) and the independently detectable probe(s), so that those bacteria that are stained with the detectable probe(s) and not the independently detectable probe(s) can be specifically determined so as to thereby specifically detect, identify and/or quantitate the bacteria of the Salmonella genus in the sample.

2. The method of Claim 1, wherein detectable and independently detectable probes are labelled with independently detectable fluorophores.

3. The method of Claim 1, wherein at least one probe is a peptide nucleic acid.

4. The method of Claim 1, wherein all probes used in the method are peptide nucleic acid.

5. The method of Claim 1, wherein at least one probe is unlabelled.

6. The method of Claim 1, wherein additional probes are used in the assay, at least one of which is a blocking probe.

7. The method of Claim 1, wherein the detectable and independently detectable probes are self-indicating probes.

8. The method of Claim 7, wherein the self-indicating probes are self-indicating beacon probes.

9. The method of Claim 1, wherein the detectable and independently detectable probes are unlabelled.

10. The method of Claim 1, wherein the sample to be tested is taken from a source selected from the group consisting of: a food, a beverage, water, a pharmaceutical product, a personal care product, a dairy product and the environment.

11. The method of Claim 1, wherein the sample to be tested is taken from a source selected from the group consisting of: a raw material, equipment, a fixture, a product and a clinical environment.

12. The method of Claim 1, wherein the sample to be tested is taken from a process used to manufacture or store food, beverages, water, pharmaceutical products, personal care products or dairy products.

13. The method of Claim 1, wherein the sample to be tested is a clinical specimen.

14. The method of Claim 1, further comprising performing a wash to thereby remove excess detectable and independently detectable probe before performing step (c).

## Patentansprüche

1. Verfahren zum spezifischen Nachweisen, Identifizieren und/oder Quantifizieren von Bakterien der Salmonella-Gattung unter anderen nachweisbaren Organismen, mit welchen eine Sonde für die Salmonella-Bakterien kreuzreagieren wird; wobei das Verfahren umfasst:
(a) das Inkontaktbringen der Probe unter geeigneten Hybridisierungsbedingungen mit einer oder mehreren nachweisbaren Sonden, welche zum Nachweisen, I-dentifizierext und/oder Quantifizieren von Bakterien der Saimonella-Gattung geeignet sind;
(b) das Inkontaktbringen der Probe unter geeigneten Hybridisierungsbedingungen mit einer oder mehreren unabhängig voneinander nachweisbaren Sonden, welche für einen oder mehreren andere nachweisbare Organismen spezifisch sind, mit welchen die nachweisbare Sonde bzw. nachweisbaren Sonden für die Salmonella-Bakterien kreuzreagieren wird bzw. werden, wobei die unabhängig voneinander nachweisbare Sonde bzw. nachweisbaren Sonden selber nicht mit den Salmonella-Bakterien kreuzreagiert bzw. kreuzreagieren;
(c) das Bestimmen einer Hybridisierung der sondierenden Nukleobasensequenz der nachweisbaren und unabhängig voneinander nachweisbaren Sonden an ihren Nukleinsäurezielsequenzen durch Nachweisen der Sonde/Zielsequenz-Komplexe in der Probe; und
(d) das Bestimmen der Gegenwart, Abwesenheit und/oder Anzahl von Organismen, welche mit sowohl der nachweisbaren Sonde bzw, den nachweisbaren Sonden als auch der unabhängig voneinander nachweisbaren Sonde bzw. den unabhängig voneinander nachweisbaren Sonden angefärbt werden, so dass jene Bakterien, welche mit der nachweisbaren Sonde bzw. den nachweisbaren Sonden und nicht der unabhängig voneinander nachweisbaren Sonde bzw. den unabhängig voneinander nachweisbaren Sonden angefärbt werden, spezifisch bestimmt werden können, so dass dabei spezifisch die Bakterien der Salmonella-Gattung in der Probe nachgewiesen, identifiziert und/oder quantifiziert werden.

2. Verfahren nach Anspruch 1, wobei nachweisbare und unabhängig voneinander nachweisbare Sonden mit unabhängig voneinander nachweisbaren Fluorophoren markiert sind.

3. Verfahren nach Anspruch 1, wobei mindestens eine Sonde eine Peptidnukleinsaure ist.

4. Verfahren nach Anspruch 1, wobei alle Sonden, welche in dem Verfahren verwendet werden, Peptidnukleinsäure sind.

5. Verfahren nach Anspruch 1, wobei mindestens eine Sonde nicht markiert ist,

6. Verfahren nach Anspruch 1, wobei zusätzliche Sonden in dem Test verwendet werden, wobei mindestens eine davon eine Blockiersonde ist.

7. Verfahren nach Anspruch 1, wobei die nachweisbaren und unabhängig voneinander nachweisbaren Sonden selbstanzeigende Sonden sind.

8. Verfahren nach Anspruch 7, wobei die selbstanzeigenden Sonden selbstanzeigende Lichtsignalsonden sind.

9. Verfahren nach Anspruch 1, wobei die nachweisbaren und unabhängig voneinander nachweisbaren Sonden nicht markiert sind.

10. Verfahren nach Anspruch 1, wobei die Probe, welche getestet wird, von einer Quelle genommen wird, welche aus der Gruppe ausgewählt ist, die besteht aus: einem Lebensmittel, einem Getränk, Wasser, einem pharmazeutischen Produkt, einem Körperpflegeprodukt, einem Molkereiprodukt und der Umgebung.

11. Verfahren nach Anspruch 1, wobei die Probe, welche getestet wird, von einer Quelle genommen wird, welche aus der Gruppe ausgewählt ist, die besteht aus: einem Rohmaterial, Ausrüstung, einer Befestigungsvorrichtung, einem Produkt und einer klinischen Umgebung.

12. Verfahren nach Anspruch 1, wobei die Probe, welche getestet wird, von einem Verfahren genommen wird, welches zur Herstellung oder Lagerung von Lebensmitteln, Getränken, Wasser, pharmazeutischen Produkten, Körperpflegeprodukten oder Molkereiprodukten verwendet wird.

13. Verfahren nach Anspruch 1, wobei die Probe, welche getestet wird, ein klinischer Prüfkörper ist.

14. Verfahren nach Anspruch 1, ferner umfassend das Durchführen einer Wäsche, um dabei überschüssige nachweisbare und unabhängig voneinander nachweisbare Sonde vor dem Durchführen von Schritt (c) zu entfernen.

## Revendications

1. Procédé de détection, d'identification et/ou de quantification spécifique de bactéries du genre Salmonella parmi d'autres organismes détectables, avec lequel une sonde contre la bactérie Salmonella réagira de façon croisée ; ledit procédé comprenant :
(a) la mise en contact de l'échantillon dans des conditions d'hybridation appropriées, avec une ou plusieurs sondes détectables pour détecter, identifier et/ou quantifier les bactéries du genre Salmonella ;
(b) la mise en contact de l'échantillon, dans des conditions d'hybridation appropriées, avec une ou plusieurs sondes détectables de façon indépendante qui sont spécifiques d'un ou plusieurs autres organismes détectables avec lesquels la/les sonde(s) détectable(s) contre la bactérie Salmonella réagiront de façon croisée, la/les sonde(s) détectable(s) de façon indépendante ne réagissant pas elles-mêmes de façon croisée avec la bactérie Salmonella ;
(c) la détermination de l'hybridation de la séquence nucléique de sondage des sondes détectables et détectables de façon indépendante à leurs séquences d'acides nucléiques cibles en détectant les complexes sonde/séquence cible dans l'échantillon ; et
(d) la détermination de la présence, de l'absence et/ou du nombre d'organismes qui sont colorés avec à la fois la/les sonde(s) détectable(s) et la/les sonde(s) détectable(s) de façon indépendante, de sorte que les bactéries qui sont colorées avec la/les sonde(s) détectable(s) et non avec la/les sonde(s) détectable(s) de façon indépendante puissent être déterminées spécifiquement afin de détecter, identifier et/ou quantifier ainsi la bactérie du genre Salmonella dans l'échantillon.

2. Procédé selon la revendication 1, dans lequel les sondes détectables et détectables de façon indépendante sont marquées avec des fluorophores détectables de façon indépendante.

3. Procédé selon la revendication 1, dans lequel au moins une sonde est un acide nucléique peptidique.

4. Procédé selon la revendication 1, dans lequel toutes les sondes utilisées dans le procédé sont des acides nucléiques peptidiques.

5. Procédé selon la revendication 1, dans lequel au moins une sonde est non marquée.

6. Procédé selon la revendication 1, dans lequel des sondes supplémentaires sont utilisées dans le test, au moins l'une d'elle étant une sonde bloquante.

7. Procédé selon la revendication 1, dans lequel les sondes détectables et détectables de façon indépendante sont des sondes auto-indicatrices.

8. Procédé selon la revendication 7, dans lequel les sondes auto-indicatrices sont des sondes à balise auto-indicatrices.

9. Procédé selon la revendication 1, dans lequel les sondes détectables et détectables de façon indépendante sont non marquées.

10. Procédé selon la revendication 1, dans lequel l'échantillon à tester est prélevé d'une source choisie dans le groupe comprenant : un aliment, une boisson, de l'eau, un produit pharmaceutique, un produit de soin personnel, un produit laitier et l'environnement.

11. Procédé selon la revendication 1, dans lequel l'échantillon à tester est prélevé à partir d'une source choisie dans le groupe comprenant un matériau brut, un équipement, une fixation, un produit et un environnement clinique.

12. Procédé selon la revendication 1, dans lequel l'échantillon à tester est prélevé dans un procédé utilisé pour produire ou stocker des aliments, des boissons, de l'eau, des produits pharmaceutiques, des produits de soin personnels ou des produits laitiers.

13. Procédé selon la revendication 1, dans lequel l'échantillon à tester est un échantillon clinique.

14. Procédé selon la revendication 1, comprenant en outre la réalisation d'un lavage pour éliminer ainsi l'excédent de sonde détectable et de sonde détectable de façon indépendante avant de réaliser l'étape (c).
